# EUROPÄISCHE PATENTSCHRIFT

(11) **EP 1 862 455 B1**
(45) Veröffentlichungstag und Bekanntmachung des Hinweises auf die Patenterteilung: **25.02.2009**
(21) Anmeldenummer: 07016889.3
(22) Anmeldetag: 16.03.2004
(51) Int. Cl.: C07D 205/08, A61K 31/397, A61P 3/06, C07C 235/74

(54) **11-((4R,6R)-4,5,6-Trihydroxy-3-(R)-hydroxy-2-(S)-hydroxy-hexylcarbamoyl)-Undekansäure**
11-((4R,6R)-4,5,6-trihydroxy-3-(R)-hydroxy-2-(S)-hydroxy-hexylcarbamoyl)-undecanoic acid
Acide 11-((4R,6R)-4,5,6-trihydroxy-3-(R)-hydroxy-2-(S)-hydroxy-hexylcarbamoyl)-undecanoique

(30) Priorität: 01.04.2003 DE 10314610
(43) Veröffentlichungstag der Anmeldung: 05.12.2007
(62) Teilanmeldung aus: 04720854.1
(73) Patentinhaber: Sanofi-Aventis Deutschland GmbH, 65929 Frankfurt am Main (DE)
(72) Erfinder: Jaehne, Gerhard, Dr., 65926 Frankfurt am Main (DE); Frick, Wendelin, Dr., 65926 Frankfurt am Main (DE); Lindenschmidt, Andreas, Dr., 65926 Frankfurt am Main (DE); Flohr, Stefanie, Dr., 65926 Frankfurt am Main (DE); Heuer, Hubert, dr., 65926 Frankfurt am Main (DE); Schaefer, Hans-Ludwig, 65926 Frankfurt am Main (DE); Kramer, Werner, Prof. Dr., 65926 Frankfurt am Main (DE); Galia, Eric, Dr., 65926 Frankfurt am Main (DE); Glombik, Heiner, Dr., 65926 Frankfurt am Main (DE)

(56) Entgegenhaltungen:
- WO-A-02/50027

## Beschreibung

Die Erfindung betrifft ein substituiertes Diphenylazetidinon, dessen physiologisch verträgliche Salze sowie physiologisch funktionelle Derivate.

Es sind bereits Diphenylazetidinone sowie deren Verwendung zur Behandlung von Hyperlipidämie sowie Arteriosklerose und Hypercholesterinämie beschrieben worden (WO 02/50027).

Der Erfindung lag die Aufgabe zugrunde, eine Verbindung zur Verfügung zu stellen, die im Gegensatz zu den in WO 02/50027 beschriebenen Verbindungen eine deutlich verbesserte Löslichkeit im oberen Dünndarm im prä-, bzw. postprandialen Zustand aufweist. Durch die verbesserte Löslichkeit der Verbindung wird eine höhere Verfügbarkeit an gelöster Substanz am Wirkort und damit eine vebesserte Wirkung gewährleistet.
Um diese verbessete Löslichkeit zu testen, wurden FaSSIF (**F**asted **S**tate **S**imulating **I**ntestinal **F**luid) und FeSSIF (**F**ed **S**tate **S**imulating **I**ntestinal **F**luid) Medien verwandt, die die pH-/Solubilisationsverhältnisse im oberen Dünndarm im prä-, bzw. postprandialen Zustand wiederspiegeln.
Der Erfindung lag weiterhin die Aufgabe zugrunde, eine Verbindung zur Verfügung zu stellen, die im Gegensatz zu den in WO 02/50027 beschriebenen Verbindungen eine erhöhte Stabilität sowohl im sauren Bereich (Magen) als auch im schwach alkalischen Bereich (Dünndarm) aufweist. Diese Eigenschaft führt zu weniger Nebenverbindungen/Spaltprodukten, die ihrerseits unerwünschte Nebenwirkungen zeigen können. Die erhöhte Stabilität im sauren Bereich ist aber auch bei der Formulierung von großem Vorteil, da keine säureresistente Kapsel/Tablette notwendig wird.

Die Erfindung betrifft daher die Verbindungen der Formel I 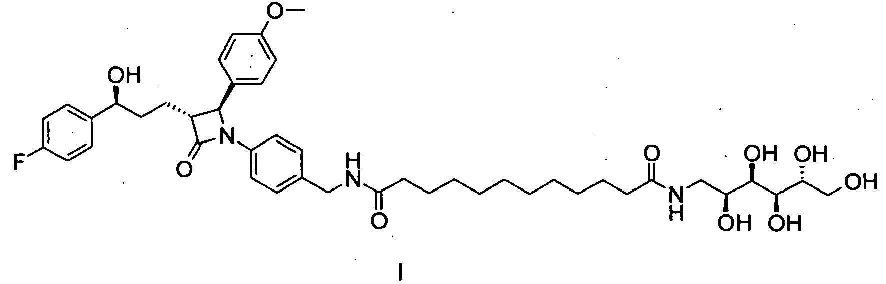 sowie deren pharmazeutisch verträgliche Salze.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindung sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindung, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäße Verbindung kann auch in verschiedenen polymorphen Formen vorliegen, z.B. als amorphe und kristalline polymorphe Form. Alle polymorphen Formen der erfindungsgemäßen Verbindung gehören in den Rahmen der Erfindung und sind ein weiterer Aspekt der Erfindung.

Nachfolgend beziehen sich alle Verweise auf "Verbindung(en) gemäß Formel I" auf Verbindung der Formel I wie vorstehend beschrieben, sowie ihre Salze, Solvate und physiologisch funktionellen Derivate wie hierin beschrieben.

Unter einem Arylrest wird ein Phenyl-, Naphthyl-, Biphenyl-, Tetrahydronaphthyl-, alpha- oder beta-Tetralon-, Indanyl- oder Indan-1-on-ylrest verstanden.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,01 mg bis 100 mg (typischerweise von 0,05 mg und 50 mg) pro Tag pro Kilogramm Körpergewicht, z.B. 0,1-10 mg/kg/Tag.

Oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine ÖI-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteter Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Als weitere Wirkstoffe für die Kombinationspräparate sind geeignet:
Alle Antidiabetika, die in der Roten Liste 2003, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus® (siehe www.lantus.com) oder HMR 1964, schnell wirkende Insuline (siehe US 6,221,633), GLP-1-Derivate wie z.B. diejenigen die in WO 98/08871 von Novo Nordisk A/S, in WO 01/04156 von Zealand oder in WO 00/34331 von Beaufour-Ipsen offenbart wurden, sowie oral wirksame hypoglykämische Wirkstoffe.
Die oral wirksamen hypoglykämischen Wirkstoffe umfassen vorzugsweise Sulphonylharnstoffe, Biguanidine, Meglitinide, Oxadiazolidindione, Thiazolidindione, Glukosidase-Inhibitoren, Hemmstoffe der Glykogenphosphorylase, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, des Glukosetransports und der Glukoserückresorption, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinyl-methoxy]phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure-{4-[(4-amino-quinazotin-2-ylamino)-methyl]-cyclohexylmethyl}-amid Hydrochlorid (CGP 71683A)), Cannabinoid Rezeptor 1 Antagonisten (siehe z.B. EP 0656354, WO 00/15609 oder WO 02/076949), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methylbenzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451)), MSH (Melanocytstimulierendes Hormon)-Agonisten, MCH (melanin-konzentrierendes Hormon) Rezeptor Antagonisten (siehe z.B. WO 03/15769), CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180), Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1 -(2-diisopropylamino-ethylcarbamoyl)-3,4-dihydro-1 H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), Hemmstoffen der 11β-HSD1 (11-beta-Hydroxysteroiddehydrogenase Typ1) (siehe z.B. WO 01/90094 oder T.Barf et al., J. Med. Chem. (2002), 45, 3813-3815), Hemmstoffen der Acetyl-CoA Carboxylase (ACC; siehe z.B. WO 99/46262), Hemmstoffen der Dipeptidylpeptidase IV (DPP-IV; siehe z.B. EP 1259246), )RXR-Modulatoren oder TR-β-Agonisten verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax^{®} ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

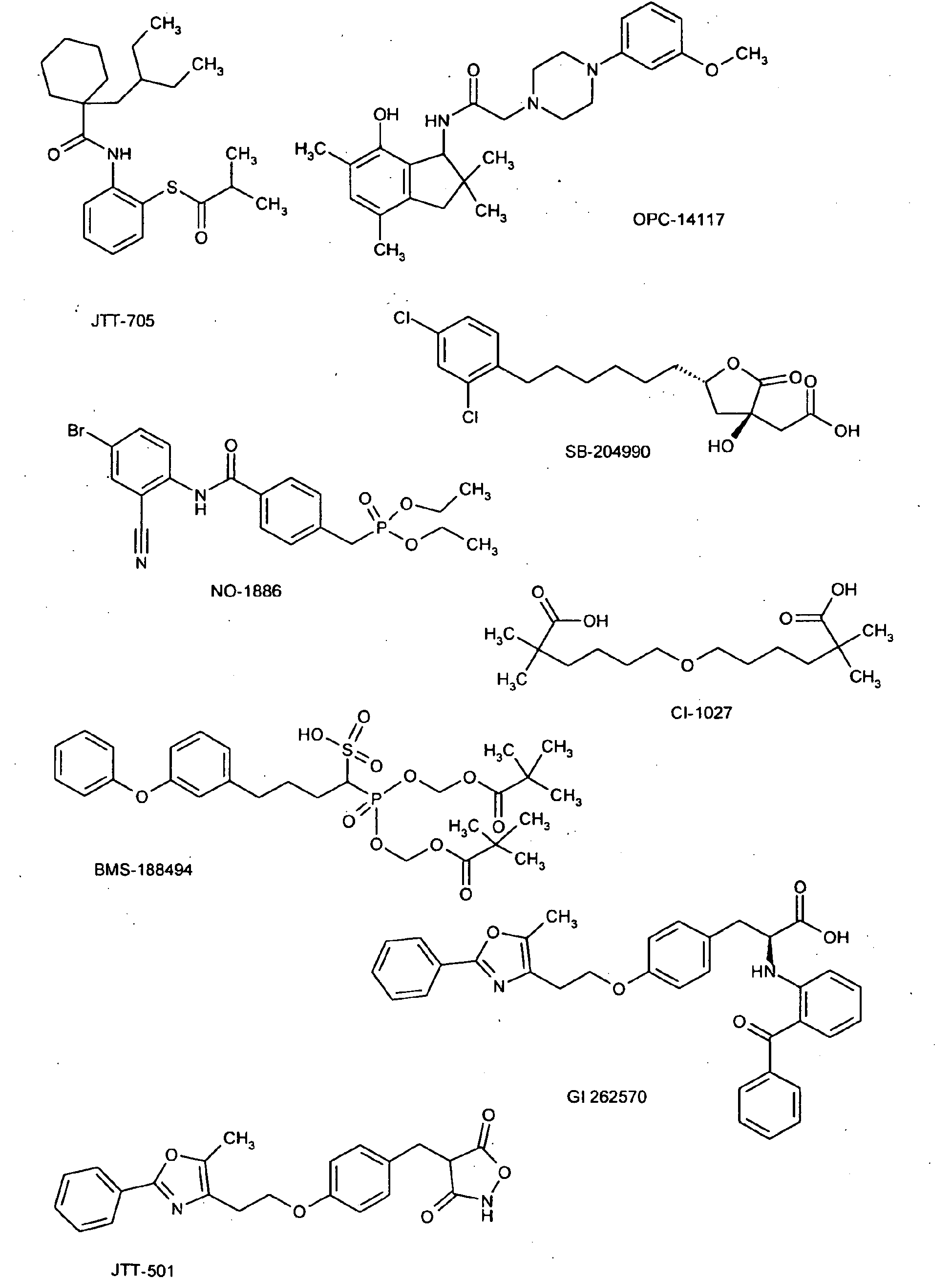

Die Erfindung betrifft weiterhin Verfahren zur Herstellung der Verbindung der Formel I.

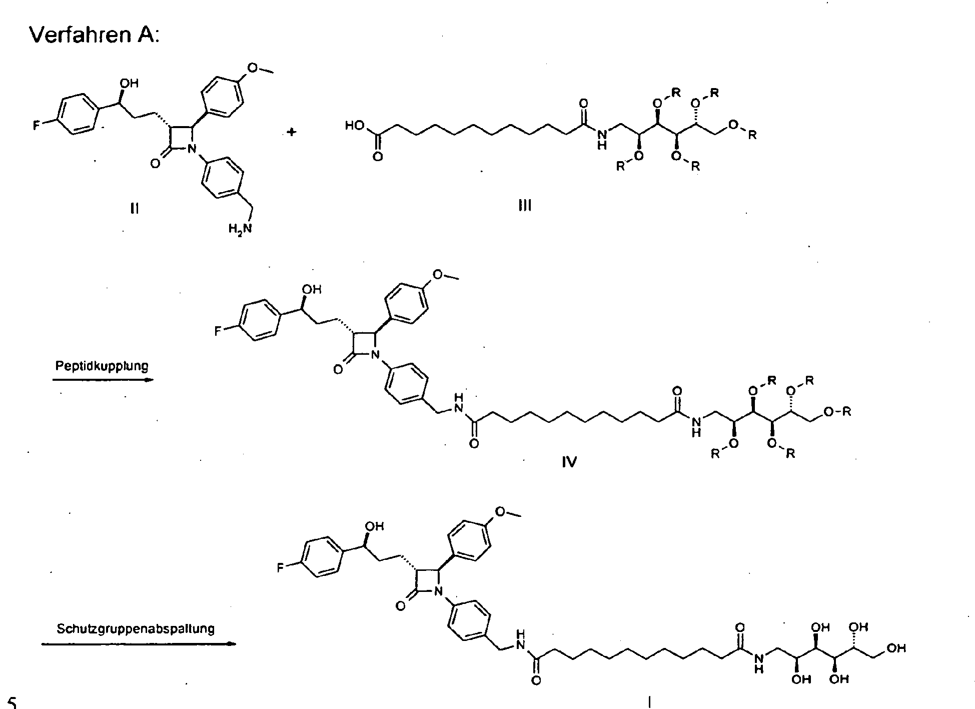

Das Verfahren A zur Herstellung der Verbindung der Formel I ist dadurch gekennzeichnet, daß man das Amin der Formel II (siehe WO 02/50027) mit dem Monoglucamid der 1,12-Dodekandicarbonsäure (Formel III), wobei die Hydroxyfunktionen des Glucaminteils mit z.B. Acylgruppen, wie z.B. Acetylgruppen, oder mit Ethergruppen, wie z.B. Benzylethergruppen, geschützt sein können, im Sinne einer Peptidkupplung zu einer Verbindung der Formel IV umsetzt. Für diese Umsetzung kann z.B. mit N-Hydroxybenzotriazol (HOBt) und N-Ethyl-N'-(3-dimethylaminopropyl)-carbodiimid (EDC) bei Raumtemperatur in z.B. Dimethylformamid (DMF) als Lösemittel gearbeitet werden. Auch andere Peptidkupplungsreagenzien und Lösemittel oder Lösemittelgemische können angewandt werden (siehe z.B. A. Speicher et al. In Journal für Praktische Chemie/Chemiker-Zeitung (1998), 340, 581-583; Y. S. Klausner und M. Bodansky, Synthesis, (1972), 453 ff; K. Ishihara et al., J. Org. Chem., 61, 4196 (1996); M. Kunishima et al., Tetrahedron 55, 13159-13170 (1999) oder auch R. C. Larock: Comprehensive Organic Transformations; VCH, New York, 1989, Seite 981 ff).

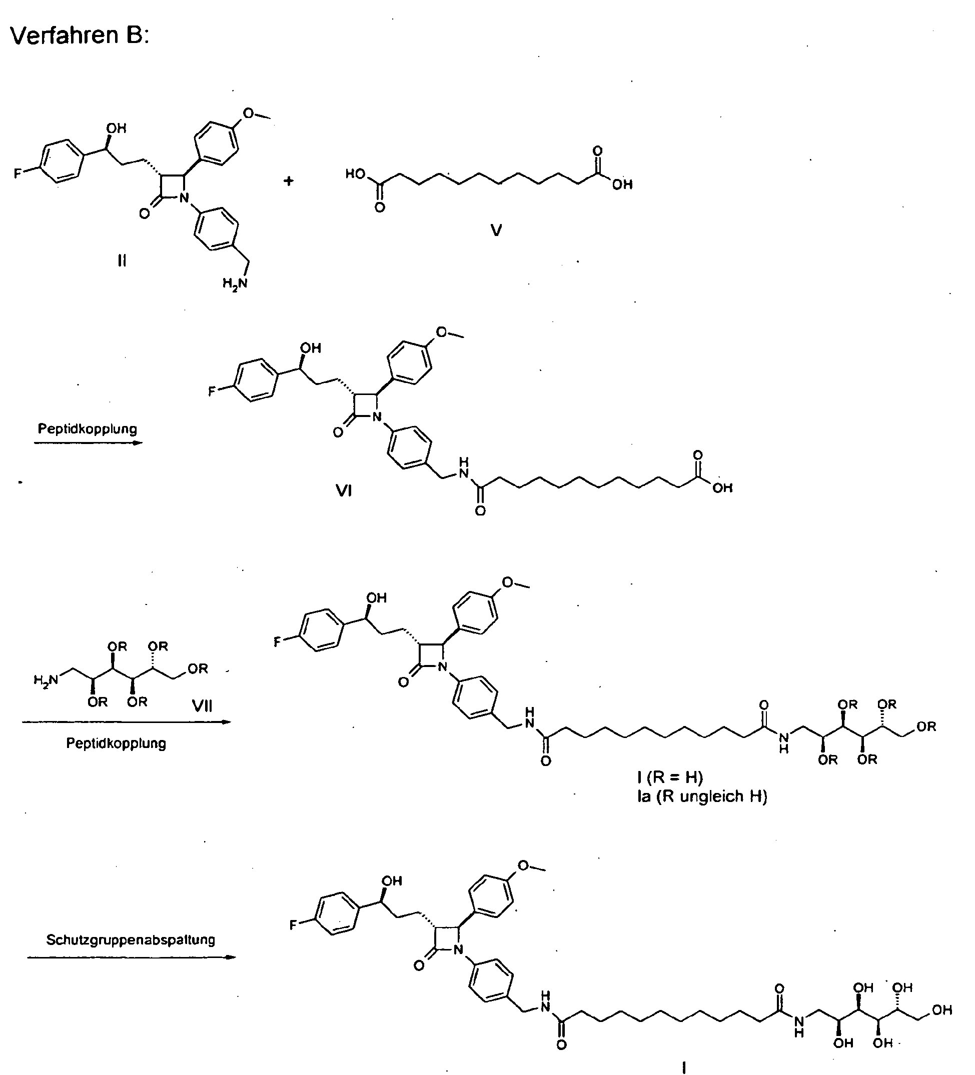

Ein weiteres erfindungsgemäßes Verfahren (B) beinhaltet den Umsatz des Amins der Formel II mit 1,12-Dodekandicarbonsäure V unter Peptidkopplungsbedingungen und die weitere Umsetzung des Produktes der Formel VI mit Glucamin VII, dessen Hydroxyfunktionen mit Schutzgruppen versehen sein können (z.B. Acetylschutzgruppen oder Benzylschutzgruppen),wiederum unter Peptidkopplungsbedingungen zur Verbindung der Formel I oder der entsprechend mit Schutzgruppen versehenen Verbindung Ia. In einem weiteren Schritt können die Schutzgruppen entweder unter schwach alkalischen Bedingungen, z. B. verdünnter wässriger Ammoniak, oder hydrogenolytisch (beim Einsatz von Benzyletherschutzgruppen) abgespalten werden, um zur Verbindung der Formel I zu gelangen.

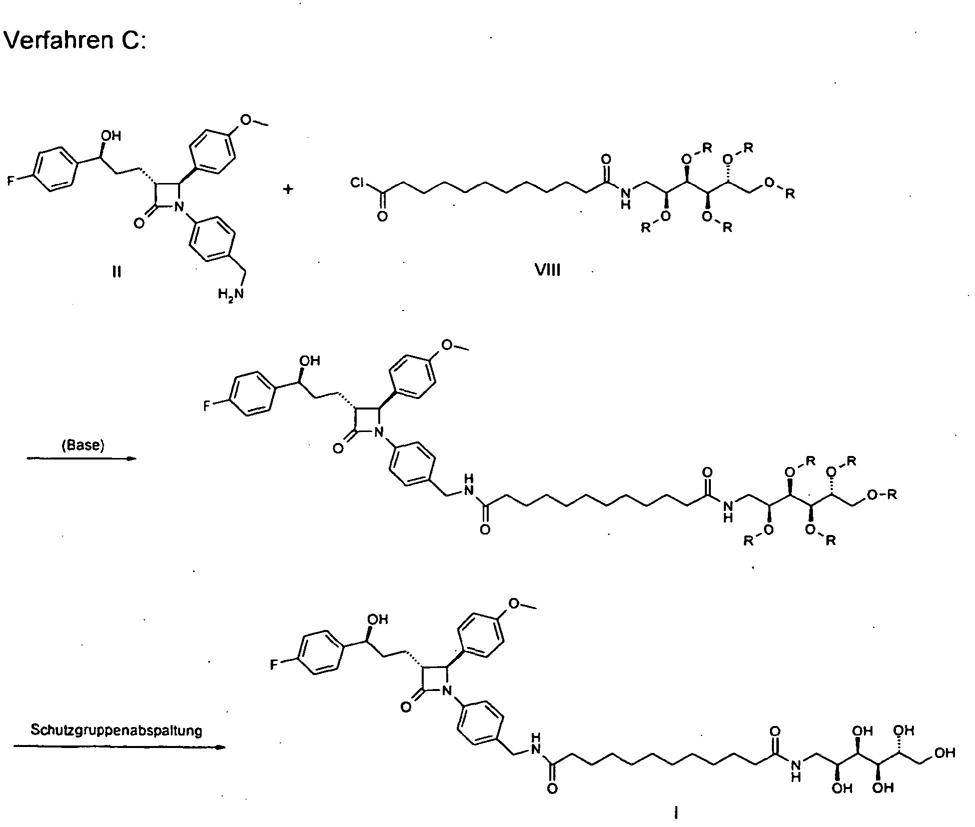

In einem weiteren erfindungsgemäßen Verfahren C, wird das Amin der Formel II mit einem Säurehalogenid, z. B. dem Chlorid, der 11-((4R,6R)-4,5,6-Trihydroxy-3-(R)-hydroxy-2-(S)-hydroxy-hexylcarbamoyl)-undekansäure VIII in z.B. Pyridin oder in Dichlormethan mit oder ohne Zusatz von Aminbase bei Raumtemperatur zur Reaktion gebracht. Die Hydroxyfunktionen des Glucaminteiles sind dabei vorteilhaft mit den oben genannten Schutzgruppen versehen und können nach der Kupplung zum Amid der Formel Ia abgespalten werden.

Weiterhin betrifft die Erfindung die Zwischenprodukte der Formeln III, IV und VIII, worin R gleich Acyl, z.B. Acetyl oder Benzoyl, ist, oder worin R gleich Aralkyl , Alkyl oder Aryl, z.B. Benzyl, ist.

### Beispiel I

Verfahren A1:
   1.) Dodekandisäuremonomethylester: 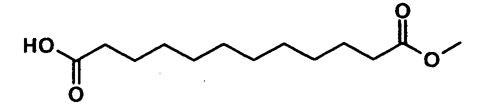 4,6 g (20 mMol) Dodekandisäure werden unter Erwärmen in 40 ml trockenem THF gelöst, mit 0,73 ml (10 mMol) Thionylchlorid langsam versetzt und 30 min bei RT gerührt. Anschließend werden 0,8 ml (20 mMol) trockenes Methanol langsam zugegeben und 4 h bei RT gerührt; der Ansatz bleibt dann 4 Tage bei RT stehen. Das DC zeigt anschließend keine weitere Umsetzung; das Reaktionsgemisch wird im Vakuum eingeengt, der Rückstand wird in Wasser verrührt (Ultraschallbad). Der Niederschlag wird abgesaugt, mit Wasser gewaschen und erneut abgesaugt. Der feuchte Rückstand wird in Dichlormethan verrührt (Ultraschallbad), über ein Faltenfilter filtriert, mit Dichlormethan gewaschen und das Filtrat wird im Vakuum eingeengt. Man erhält den Dodekandisäuremonomethylester (3,09 g) in einer Ausbeute von 63%. MG: 244,34; MS: 245,4 (M+H⁺).
   2.) Synthese von 11-((4R,6R)-4,5,6-Trihydroxy-3-(R)-hydroxy-2-(S)-hydroxy-hexylcarbamoyl)-undekansäuremethylester: 3,07 g (12,6 mMol) Dodekandisäuremonomethylester werden bei Raumtemperatur in 30 ml trockenem DMF gelöst, mit 2,2 g (12,1 mMol) Glucamin, 1,9 g (12,4 mMol) HOBt und 2,4 g (12,5 mMol) EDC versetzt und 6 h bei RT gerührt. Man lässt über Nacht bei RT stehen. Am folgenden Tag zeigt das DC vollständige Umsetzung. Das Reaktionsgemisch wird im Vakuum eingeengt und am Hochvakuum getrocknet. Der Rückstand wird in Wasser verrührt (Ultraschallbad), abgesaugt, mit Wasser gewaschen und abgesaugt. Das feuchte Rohprodukt wird in Dichlormethan verrührt, abgesaugt, mit Dichlormethan gewaschen und getrocknet. Man erhält 11-((4R,6R)-4,5,6-Trihydroxy-3-(R)-hydroxy-2-(S)-hydroxy-hexylcarbamoyl)-undekansäuremethyl ester. 4,45 g (90% Ausbeute). MG: 407,51; MS: 408,20 (M+H⁺).
   3.) Synthese von 11-((4R,6R)-4,5,6-Trihydroxy-3-(R)-hydroxy-2-(S)-hydroxy-hexylcarbamoyl)-undekansäure (III; R = H): 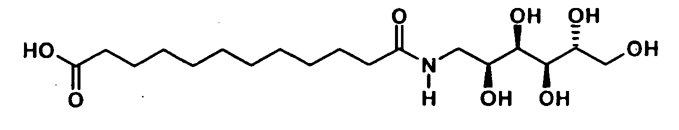 4,45 g (10,9 mMol) 11-((4R,6R)-4,5,6-Trihydroxy-3-(R)-hydroxy-2-(S)-hydroxy-hexylcarbamoyl)-undekansäuremethy ester werden bei Raumtemperatur in 75 ml trockenem Ethanol suspendiert, mit 25 ml Wasser und 2,2 g KOH (85%ig) (33 mMol) versetzt. Nach 2 h Rühren bei 80°C zeigt das DC vollständige Umsetzung. Das Reaktionsgemisch wird im Vakuum eingeengt; der Rückstand wird in Wasser gelöst und mit konz. Salzsäure angesäuert. Das ausgefallene Rohprodukt wird abgesaugt, mit Wasser gewaschen und abgesaugt. Das feuchte Rohprodukt wird aus ca. 100 ml Ethanol umkristallisiert, heiß filtriert und im Eisbad ausgefällt. Man saugt den Niederschlag ab, wäscht ihn mit Ethanol und trocknet ihn. Man erhält 2,2 g (51 %) 11-((4R,6R)-4,5,6-Trihydroxy-3-(R)-hydroxy-2-(S)-hydroxy-hexylcarbamoyl)-undekansäure. MG: 393,48; MS: 394,28 (M+H⁺).
   4.) Synthese von Dodekandisäure 4-[(2S,3R)-3-[(S)-3-(4-fluoro-phenyl)-3-hydroxypropyl]-2-(4-methoxy-phenyl)-4-oxo-azetidin-1-yl]-benzylamid ((2S,3R,4R,5R)-2,3,4,5,6-pentahydroxy-hexyl)-amid (I): 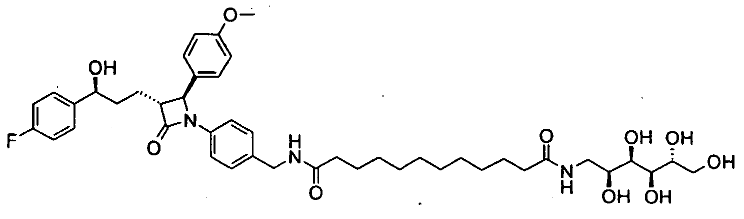 0,63 g (1,45 mMol) des Benzylamins II (Herstellung siehe DE10064398) und 0,65 g (1,65 mMol) des Disäuremonoamids (siehe oben) werden unter leichtem Erwärmen in 15 ml trockenem DMF gelöst, mit 0,25 g (1,63 mMol) HOBt und 0,31 g (1,67 mMol) EDC versetzt und 4 h bei RT gerührt. Die Reaktionsmischung bleibt über Nacht bei RT stehen. Am nächsten Morgen zeigt das DC vollständige Umsetzung an. Das Reaktionsgemisch wird im Vakuum eingeengt und der Rückstand im Hochvakuum getrocknet. Der Rückstand wird in Wasser verrührt (Ultraschallbad), abgesaugt, mit Wasser gewaschen und abgesaugt. Das Rohprodukt wird aus Isopropanol umkristallisiert. Das Kristallisat wird noch mit Wasser verrührt, abgesaugt und getrocknet. Man erhält 0,38 g (32%) Dodekandisäure 4-[(2S,3R)-3-[(S)-3-(4-fluoro-phenyl)-3-hydroxy-propyl]-2-(4-methoxy-phenyl)-4-oxo-azetidin-1-yl]-benzylamid ((2S,3R,4R,5R)-2,3,4,5,6-pentahydroxy-hexyl)-amid (I). MG: 809,97; MS: 810,49 (M+H⁺).
Verfahren A2:
   1.) 11-((2S,3R,4R,5R)-2,3,4,5,6-Pentaacetoxy-hexylcarbamoyl)-undekansäure (III; R = Acetyl): 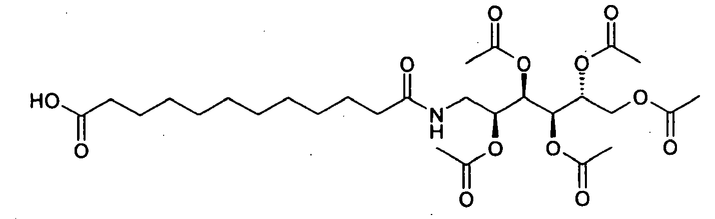 0,4 g 11-((4R,6R)-4,5,6-Trihydroxy-3-(R)-hydroxy-2-(S)-hydroxy-hexylcarbamoyl)-undekansäure (III; R = H) werden bei Taumtemperatur mit 3 ml trockenem Pyridin und 3 ml Essigsäureanhydrid versetzt und 4h bei Raumtemperatur gerührt.. Nach beendeter Reaktion wird das Reaktionsgemisch mit Wasser versetzt und im Vakuum eingeengt. Der Rückstand wird mit wenig Wasser verrührt und filtriert. Der Filterrückstand wird mit Wasser gewaschen und anschließend im Vakuum getrocknet. Man erhält 0.56 g 11-((2S,3R,4R,5R)-2,3,4,5,6-Pentaacetoxy-hexylcarbamoyl)-undekansäure. MG: 603,66; MS: 604,22 (M+H⁺).
   2.) Acetic acid (2R,3R,4R,5S)-2,3,4,5-tetraacetoxy-6-(11-{4-[(2S,3R)-3-[(S)-3-(4-fluoro-phenyl)-3-hydroxy-propyl]-2-(4-methoxy-phenyl)-4-oxo-azetidin-1-yl]-benzylcarbamoyl}-undecanoylamino)-hexyl ester (IV; R = Acetyl): 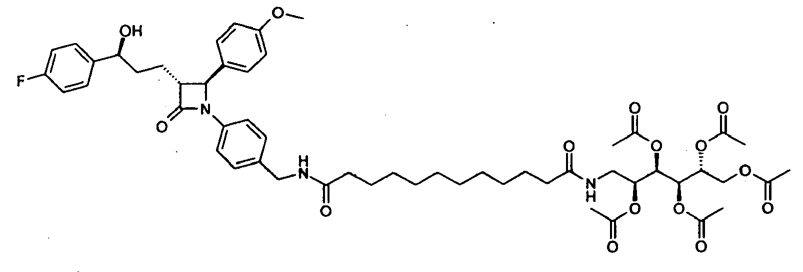 87 mg des Amins II werden bei Raumtemperatur in 3 ml trockenem Dimethylformamid gelöst und mit 120 mg der oben beschriebenen Carbonsäure, 31 mg N-Hydroxy-Benzotriazol und 39 mg N-Ethyl-N'-(3-dimethylaminopropyl)-carbodiimid versetzt. Die Reaktionsmischung wird über Nacht bei Raumtemperatur gerührt und danach im Vakuum eingeengt. Der Rückstand wird in Essigsäureethylester aufgenommen, die organische Phase mit Wasser gewaschen und über Magnesiumsulfat getrocknet. Anschließend wird filtriert und das Filtrat im Vakuum eingeengt. Man erhält 90 mg Acetic acid (2R,3R,4R,5S)-2,3,4,5-tetraacetoxy-6-(11-{4-[(2S,3R)-3-[(S)-3-(4-fluoro-phenyl)-3-hydroxy-propyl]-2-(4-methoxy-phenyl)-4-oxo-azetidin-1-yl]-benzylcarbamoyl}-undecanoylamino)-hexyl ester. MG: 1020,16.
   3.) Dodekandisäure 4-[(2S,3R)-3-[(S)-3-(4-fluoro-phenyl)-3-hydroxy-propyl]-2-(4-methoxy-phenyl)-4-oxo-azetidin-1-yl]-benzylamid ((2S,3R,4R,5R)-2,3,4,5,6-pentahydroxy-hexyl)-amid (I): 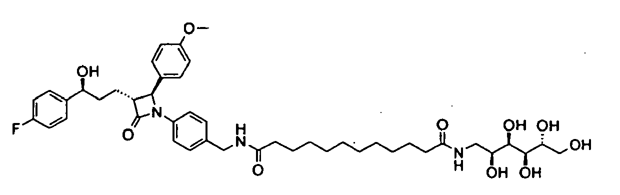 90 mg der oben beschriebenen Verbindung werden mit Guanidin in einer Mischung aus Ethanol und Dichlormethan behandelt. Man erhält das Glucaminderivat I mit dem MG 809,97.
Verfahren B:
   1.) 11-{4-[(2S,3R)-3-[(S)-3-(4-Fluoro-phenyl)-3-hydroxy-propyl]-2-(4-methoxy-phenyl)-4-oxo-azetidin-1-yl]-benzylcarbamoyl}-undekansäure (VI): 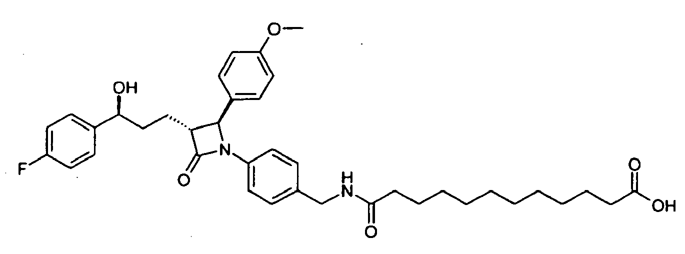 Zu einer Lösung aus 371 mg Dodekandisäure, 63 µl Diisopropylcarbodiimid, 55 mg Hydroxybenzotriazol in 2 ml Dimethylformamid wird eine Lösung aus 70 mg des Amins I, 23 µl Triethylamin in 1 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1 % Trifluoressigsäure) = 80/20 -> 10/90) getrennt. Man erhält das Produkt mit einem Molekulargewicht von 646.81 (C₃₈H₄₇F₁N₂O₆); MS (ESI) 647.35 (M + H⁺)
   2.) Dodekandisäure 4-[(2S,3R)-3-[(S)-3-(4-fluoro-phenyl)-3-hydroxy-propyl]-2-(4-methoxy-phenyl)-4-oxo-azetidin-1-yl]-benzylamid ((2S,3R,4R,5R)-2,3,4,5,6-pentahydroxy-hexyl)-amid (I): 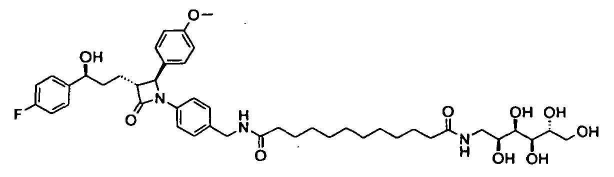 Wie vorn für andere Kupplungsreaktionen beschrieben, liefert der Umsatz der Säure VI mit Glucamin (VII; R = H) und HOBt/EDC in DMF die Verbindung I (R = H).
      Wird statt Glucamin ein geschütztes Glucaminderivat, z.B. VII (R = Acetyl) eingesetzt erhält man die Verbindung Ia mit R = Acetyl.
Verfahren C:
   1.) Acetic acid (2R,3R,4R,5S)-2,3,4,5-tetraacetoxy-6-(11-chlorocarbonyl-undecanoylamino)-hexyl ester (VIII; R = Acetyl): 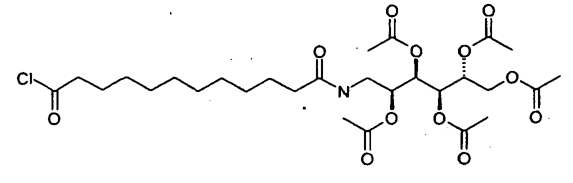 Die Verbindung der Formel III (R = Acetyl) wird in Tetrahydrofuran gelöst und langsam mit Thionylchlorid versetzt; es wird 1 h bei Raumtemperatur gerührt. Danach wird die Reaktionslösung im Vakuum eingeengt und das Rohpodukt in die nächste Stufe eingesetzt.
   2.) Acetic acid (2R,3R,4R,5S)-2,3,4,5-tetraacetoxy-6-(11-{4-[(2S,3R)-3-[(S)-3-(4-fluoro-phenyl)-3-hyd roxy-propyl]-2-(4-methoxy-phenyl)-4-oxo-azetid in-1-yl]-benzylcarbamoyl}-undecanoylamino)-hexyl ester (IV; R = Acetyl): 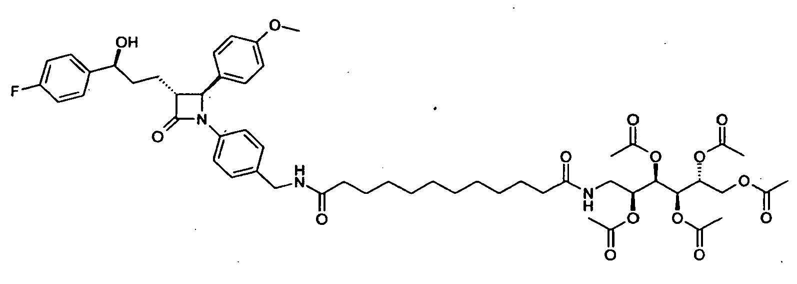 Das oben dargestellte Säurechlorid wird in einer Mischung aus Pyridin und Dichlormethan bei Raumtemperatur mit dem Amin I versetzt und über Nacht bei Raumtemperatur gerührt. Die Aufarbeitung liefert das Amid IV mir R = Acetyl.

Die erfindungsgemäße Verbindung der Formel I wurde mit der nachfolgend beschriebenen Methode auf ihre Wirkung geprüft:

### Beeinflussung der Cholesterolabsorption + ³H- Taurocholsäureausscheidung anhand der fäkalen Ausscheidung an der Maus, Ratte oder Hamster

NMRI- Mäuse, Wistar-Ratten, oder Golden Syrian Hamster (in Gruppen von n=4-6) werden unter Standarddiät (Altromin, Lage (Lippe)) in Stoffwechselkäfigen gehalten. Am Nachmittag vor Gabe der radioaktiven Tracer (¹⁴C-Cholesterol) werden die Tiere nüchtern gesetzt und auf Gitterroste adaptiert.

Zusätzlich werden die Tiere werden 24 Stunden vor der peroralen Applikation der Testmahlzeit (¹⁴C-Cholesterol in Intralipid® 20, Pharmacia-Upjohn) mit ³H-TCA (Taurocholic acid) s.c. gelabelt (z.b. 1 µCi/Maus bis 5 µCi/Ratte)

Cholesterolabsorptionstest: 0,25 ml/Maus Intralipid ® 20 (Pharmacia- Upjohn) ((Spikung mit 0,25 µCi ¹⁴C-Cholesterol in 0,1 mg Cholesterol) werden peroral mit der Schlundsonde verabreicht.

Testsubstanzen werden getrennt in 0,5 %/ (Methylcellulose (Sigma)/5% Solutol (BASF, Ludwigshafen) oder geeignetem Vehikel angesetzt.
Das Applikationsvolumen der Testsubstanz beträgt 0,5 ml/Maus. Die Testsubstanz wird unmittelbar vor der Testmahlzeit (Intralipid mit ¹⁴C-Cholesterol-label) (Cholesterolabsorptionstest) appliziert.

Der Kot wird über 24 h gesammelt: die fäkale Elimination von ¹⁴C-Cholesterol und ³H Taurocholsäure (TCA) nach 24 Std. wird bestimmt.

Die Lebern werden entnommen, homogenisiert und Aliquots im Oximaten (Model 307, Packard) verbrannt zur Bestimmung der aufgenommenn/resorbierten Menge an ¹⁴C-Cholesterol.

### Auswertung:

### Kotproben:

Gesamtgewicht bestimmen, mit Wasser auf definiertes Volumen auffüllen, dann homogenisieren, Aliquot eintrockenen und im Oximat (Model 307, Packard zur Verbrennung von radioaktiv gelabelten Proben) verbrennen: Die Menge von radioaktiv ³H- H20 und ¹⁴C- CO2 wird hochgerechnet auf die ausgeschiedene Menge an ³H-Taurocholsäure bzw. ¹⁴C-Cholesterol (Dual-Isotopen-Technik ). Die ED₂₀₀-Werte werden als Dosis aus einer Dosiswirkungskurve interpoliert als diejenige Dosen, die die Auscheidung an TCA bzw. Cholesterol verdoppeln, bezogen auf eine zeitgleich behandelte Kontrollgruppe.

### Leberproben:

Die aufgenommene Menge von ¹⁴C-Cholesterols in die Leber wird bezogen auf die applizierte Dosis. Die ED₅₀ Werte werden interpoliert aus einer Dosiswirkungskurve als diejenige Dosis, die die Aufnahme von ¹⁴C- Cholesterol in die Leber halbiert (50%), bezogen auf eine Kontrollgruppe

Der folgende ED₅₀-Wert belegt die Aktivität der erfindungsgemäßen Verbindung der Formel I

| Beispiel Nr. | ED₅₀ (Leber) [mg/Maus] |
|---|---|
| I | 0.005 |

Aus der Tabelle ist abzulesen, daß die Verbindung der Formel I eine sehr gute Cholesterin senkende Wirkung besitzt.

Die Löslichkeit der Verbindung I sowie die der Vergleichsverbindung V1 wurde wie folgt getestet:

Als Vergleichsverbindung wurde die strukturell ähnlichste Verbindung aus WO02/50027ausgewählt: 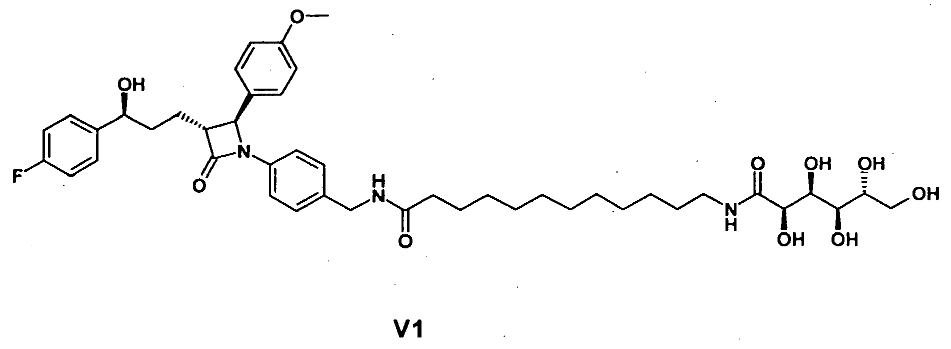 0,5 mg der zu testenden Verbindung wurden in ein Eppendorf-Cap genau eingewogen und mit 0,5 ml des jeweiligen Lösungsmittels (wäßriger Puffer) versetzt. Das Eppendorf-Cap wurde dann in einen Thermomixer eingebracht und bei 25°C 4 Stunden lang mit 1400 U/min geschüttelt.
Dann gab man das Eppendorf-Cap in eine Zentrifuge. Nach dem Zentrifugieren verwendete man ein Aliquot des Überstands zur Bestimmung der gelösten Menge mittels der HPLC/UV-Analyse. Die folgende Tabelle zeigt die hierbei erzielten Ergebnisse:

| | Beispiel 1 | V1 |
|---|---|---|
| pH-Verhältnisse | Löslichkeit in µg/ml | Löslichkeit in µg/ml |
| Wasser (pH) | 3 (6,8) | < 1 |
| pH 1,2 | 3 | < 1 |
| pH 4,5 | 4 | < 1 |
| pH 6,8 | 2 | < 1 |
| pH 8,0 | 2 | < 1 |
| FaSSIF | 28 | 5 |
| FeSSIF | 454 | 18 |

In den physiologischen Lösemitteln FaSSIF und FeSSIF (Zusammensetzung und Herstellungsverfahren siehe (Physiologically based dissolution tests - Experiences with poorly soluble drugs) Auflösungsversuche auf physiologischer Grundlage - Erfahrungen mit schlecht löslichen Arzneimitteln), Januar 2000, Shaker-Verlag, ISBN:3-8265-6962-8) wurde die Löslichkeit von Beispiel 1 mit 28 und 454 µg/ml bestimmt, während die entsprechenden Werte für V1 5 bzw. 18 µg/ml betrugen. Diese signifikant unterschiedliche Löslichkeit konnte auch bei einer Wiederholung der Tests bestätigt werden (43/290 µg/ml gegenüber 6/20 µg/ml).
Die erfindungsgemäße Verbindung der Formel I ist somit 6- bis 16-fach besser löslich als die Vergleichsverbindung der Formel V1. Die erfindungsgemäße Verbindung der Formel I besitzt somit eine höhere Verfügbarkeit in gelöster Form am Wirkort. Auch gegebenenfalls höhere Dosen können hier anders als die schlechter löslichen Substanzen noch vollständig zur Interaktion mit dem entsprechenden Transportsystem zur Verfügung gestellt werden. Ausgehend von einem zur Verfügung stehenden Volumen von 250ml (Biopharmaceutical Classification System) sind Dosen von bis zu ~100mg löslich, wohingegen von V1 bestenfalls nur Dosen im Bereich von 5mg löslich wären (im schlechtesten Fall sogar nur: 1,25mg).

Die Stabilität der Verbindung I sowie die der Vergleichsverbindung V1 in Lösung wurde wie folgt getestet:

Die Stabilität von gelöster Verbindung I sowie gelöster V1 wurde in wäßrigen Puffern im pH-Bereich 1,2 - 8,0 bestimmt. 1 mg der jeweiligen Verbindung wurde in einen 5-ml-Meßkolben eingewogen. Zur Lösung der Substanz wurde eine geringe Menge Acetonitril verwendet. Dann wurde mit dem wäßrigen Puffer bis zur Marke aufgefüllt. Nach dem Zentrifugieren der ausgefällten Verbindung wurde der klare Überstand 24 Stunden lang bei 37°C auf Stabilität in Lösung geprüft. Die Auswertung der Proben erfolgte mittels HPLC/UV. Die mit Beispiel I und V1 erzielten Ergebnisse sind der folgenden Tabelle zu entnehmen:

| | Beispiel I | V1 |
|---|---|---|
| pH-Verhältnisse | Prozentuale Zunahme der Fläche der Verunreinigungen | Prozentuale Zunahme der Fläche der Verunreinigungen |
| pH 1,2 | 4,9 | 13,3 |
| pH 6,8 | 0 | 0,5 |
| pH 8,0 | 0,2 | 4,6 |

Die erfindungsgemäße Verbindung der Formel I ist somit abhängig vom ph-Wert um mindestens 2,7-fach stabiler als V1 und bildet daher weinger Nebenprodukte als V1. Geringere Mengen an systemisch wirkenden Nebenprodukten bedeuten ein geringeres Potential für unerwünschte Nebenwirkungen.

## Patentansprüche

1. Zwischenprodukt der Formel

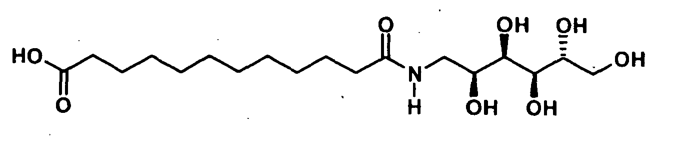

## Claims

1. An intermediate of the formula I

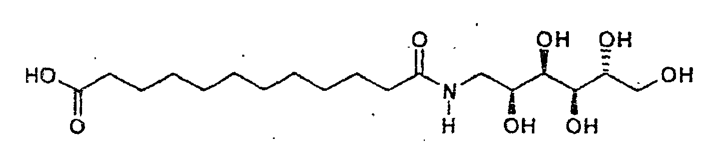

## Revendications

1. Intermédiaire de formule
